# EUROPÄISCHE PATENTSCHRIFT

(11) **EP 0 421 008 B1**
(45) Veröffentlichungstag und Bekanntmachung des Hinweises auf die Patenterteilung: **02.06.1993**
(21) Anmeldenummer: 89118369.1
(22) Anmeldetag: 04.10.1989
(51) Int. Cl.: A61F 2/36

(54) **Femur-Hüftgelenkendoprothese**
Femoral hip joint endoprosthesis
Partie fémorale pour endoprothése d'articulation de la hanche

(43) Veröffentlichungstag der Anmeldung: 10.04.1991
(73) Patentinhaber: S + G IMPLANTS GMBH, D-23556 Lübeck (DE)
(72) Erfinder: Henssge, Ernst Joachim, Prof. Dr. med., D-2400 Lübeck (DE); Köller, Wolfgang, Dr., D-2400 Lübeck 16 (DE); Dufek, Pavel, Dr. med., D-2400 Lübeck (DE); Scholz, Jörg, Dr. med., D-1000 Berlin 31 (DE)
(74) Vertreter: Eisenführ, Speiser & Partner

(56) Entgegenhaltungen:
- EP-A- 0 295 200
- WO-A-85/05027
- US-A- 4 595 393

## Beschreibung

Die Erfindung betrifft eine Femur-Hüftgelenkendoprothese mit einem zu seinem einen Ende hin konisch auslaufenden, anatomisch geformten Stiel und mit einer an diesem gehaltenen Gelenkkugel für eine Gelenkpfanne. Eine solche Prothese ist aus der US-A-4 595 393 bekannt.

Die meisten bekannten Hüftgelenkendoprothesen sind massiv geschmiedet oder gegossen und weisen daher je nach dem spezifischen Gewicht der eingesetzten Werkstoffe ein relativ hohes Gewicht auf. Derartige Hüftgelenkendoprothesen bestehen aus einem Stiel zur Einführung in die Markhöhle des Oberschenkels und einem Halsteil, auf das entweder ein Kopfteil aufgesteckt oder aufgeschweißt wird.

Der Erfindung liegt die Aufgabe zugrunde , eine Femur-Hüftgelenkendoprothese ohne Halsteil und von geringem Gewicht, jedoch mit einer großen Variabilität in der Wahl der Aufsteckverbindungen für den Kopf oder die Gelenkkugel zu schaffen.

Diese Aufgabe wird durch die in den Patentansprüchen 1 und 2 angegebenen Merkmale gelöst.

Bei einer derart erfindungsgemäß ausgebildeten Endoprothese wird gänzlich auf das sonst bekannterweise vorgesehene Halsteil verzichtet; die Endoprothese ermöglicht eine große Variabilität in der Wahl der Aufsteckverbindungen. Der Chirurg verankert die Endoprothese derart in dem Femur-Schaft, daß das obere Ende der Endoprothese an der Resektionsfläche des Knochens liegt und anschliessend die beliebige Koppelung mit verschiedenen Doppelkonus-Steckverbindungen ermöglicht. Sowohl im Falle der Erstoperation als auch bei möglichen Revisionsoperationen stellt die durch diese Endoprothese realisierte Trennung von Stiel und Hals einer Hüftgelenkendoprothese für den operierenden Arzt einen großen Gewinn dar. Wechseleingriffe einer Komponente werden so wesentlich erleichtert.

Die äußere Form der Endoprothese entspricht den bekannten, adaptierten Formen; sie kann glatt ausgeführt oder mit strukturierter Oberfläche versehen sein.

Durch die Innenhohlraumausgestaltung im oberen Bereich des Stiels der Endoprothese wird erreicht, daß diese gegenüber den bekannten Prothesen ein geringeres Gewicht aufweist. Die Endoprothese wird dadurch gewichtsmäßig leichter; sie kommt dadurch der Leichtbaukonstruktion des Knochens entgegen. Durch die oval-konische Öffnung des Haltestutzens des Stiels sind verschieden gestaltete Doppelkonus-Steckverbindungen einsetzbar, d.h. die Verwendung von Doppelkonen mit unterschiedlichen Längen und Durchmessern ist möglich und wird vereinfacht.Durch die ovale Form des Innenraumquerschnitts des Haltestutzens und des Doppelkonus der Steckverbindung werden ungewollte Verdrehungen aufgesteckter Doppelkonen bei der Implantation unmöglich gemacht. Hinzu kommt, daß durch die Hohlkammerausbildung im oberen Ende des Stiels der Endoprothese und durch die Einziehung des Haltestutzens für den Doppelkonus in den Stielhohlraum eine hohe Elastizität erhalten wird,ohne daß die Festigkeit des Stiels in seinem oberen Bereich vermindert wird.

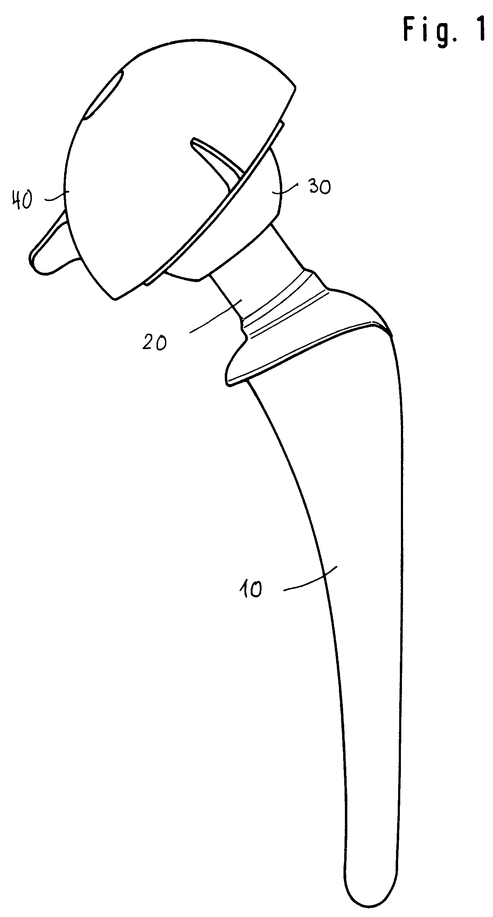

Ausführungsbeispiele der Erfindung werden nachstehend anhand der Zeichnung näher erläutert. Es zeigen
- Fig. 1: eine Vorderansicht einer Hüftgelenkendoprothese,
- Fig. 2: einen senkrechten Längsschnitt durch einen Femur-Schaft mit eingesetztem, eine Doppelkonus-Steckverbindung aufweisenden Schaft,
- Fig. 3: einen Längsschnitt eines in einen Femur-Schaft eingesetzten, eine Doppelkonus-Steckverbindung tragenden Stiels in einer gegeüber dem Stiel gem.Fig.2 kürzeren Länge,
- Fig. 4: eine Seitenansicht zu Fig.3,
- Fig. 5: einen waagerechten Schnitt gem. Linie V-V- in Fig.3 und
- Fig. 6: einen waagerechten Schnitt gem. Linie VI-VI in Fig.3.

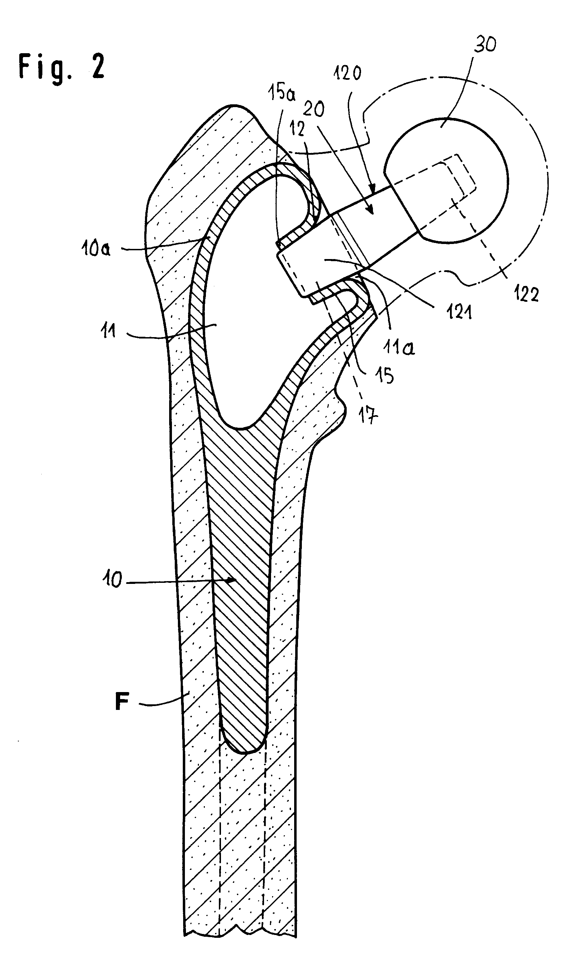

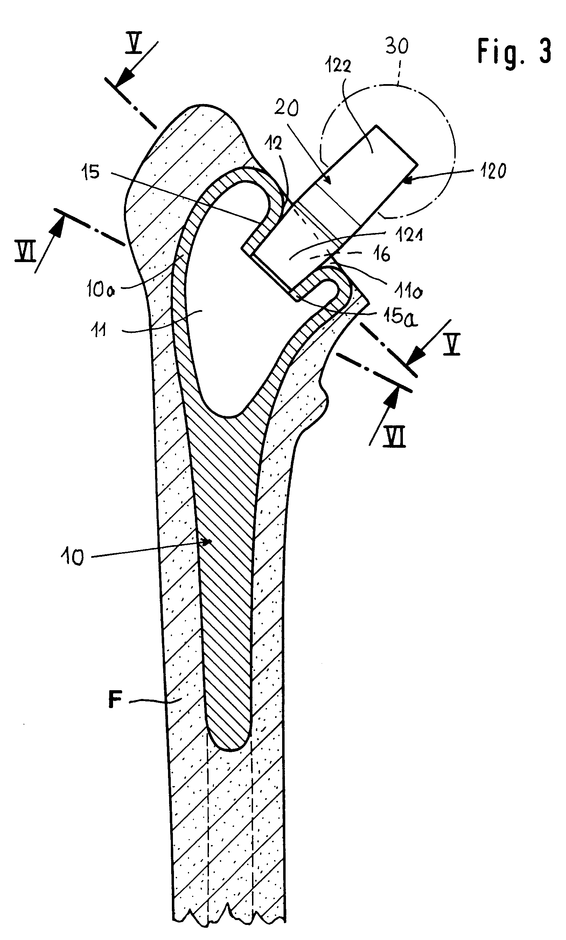

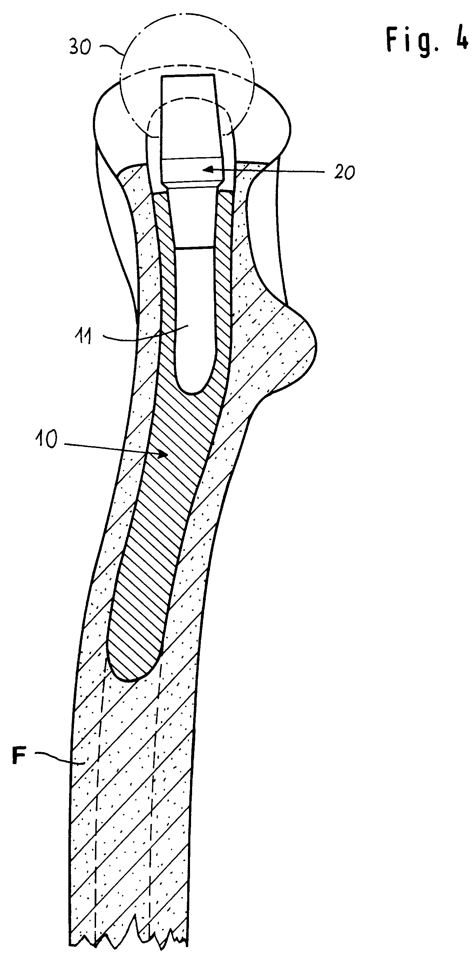

Die Femur-Hüftgelenkendoprothese besteht aus einem Stiel 10, einer Gelenkkugel 30 und einer Gelenkpfanne 40, wobei die Gelenkkugel über eine Doppelkonus-Steckverbindung 20 mit dem Stiel 10 verbunden ist. In den Fig.2,3 und 4 ist ein Femur-Schaft, in den der Stiel 10 der Endoprothese implantiert ist, mit F bezeichnet.

In dem oberen Ende 10a des Stieles 10 ist in diesem ein Hohlraum 11 ausgebildet, der entsprechend der jeweils gewählten anatomischen Form des Stiels 10 eine entsprechende Form und Abmessungen aufweist. Nach Möglichkeit soll sich dieser Hohlraum 11 so weit in den Stiel 10 erstrecken,soweit dies möglich ist, ohne daß jedoch die Festigkeit des Stiels in irgendeiner Weise beeinträchtigt oder geschwächt wird.

Der die obere Hohlraumöffnung 11a des Hohlraumes 11 begrenzende, umlaufende Rand 12 des Stiels 10 ist unter Ausbildung eines halsartigen und rohrförmigen Haltestutzens 15 in den Hohlraum 11 eingezogen (Fig.2,3 und 4). Die Länge des Haltestutzens 15 ist dabei so gewählt, daß ein fester Sitz eines in den Haltestutzen 15 eingesetzten Doppelkonus 120 einer Steckverbindung 20 gewährleistet ist, ohne daß dabei Innenhohlraum verlorengeht oder die den Hohlraum begrenzende Wand in ihrer Stabilität beeinträchtigt wird.

Der Haltestutzen 15, der zur Aufnahme des Doppelkonus 120 dient, weist nach einer Ausführungsform der Erfindung einen sich zu seinem freien Ende 15a hin konisch verlaufenden Innenhohlraum 17 auf, so daß der Haltestutzen 15 ein beliebig gestaltetes Außenprofil oder eine beliebig gestaltete Außenwandform aufweisen kann,um beispielsweise eine zusätzliche Abstützung des Haltestutzens 15 zu der den Hohlraum 11 begrenzenden Innenwandfläche vornehmen zu können.

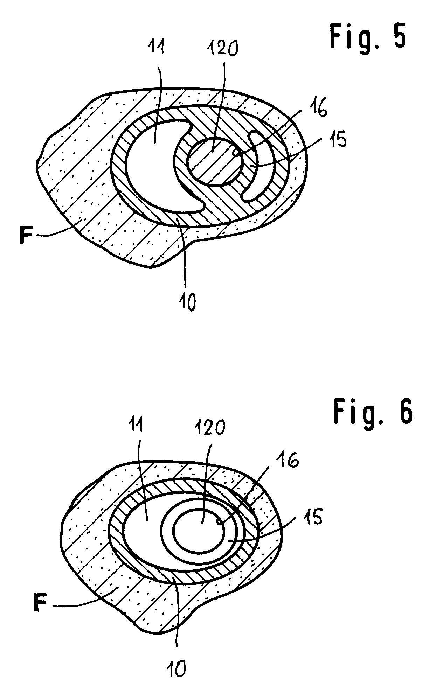

Der Haltestutzen 15 weist ferner einen ovalen Innenquerschnitt auf,der bei 16 in Fig.5 und 6 angedeutet ist. Auch der Doppelkonus 120 weist zumindest in seinem Haltestutzeneinführungsendbereich 121 eine Formgebung und insbesondere Querschnittsform auf, die der ovalen Innenquerschnittsform des Haltestutzens 15 entspricht, so daß der Doppelkonus 120 ebenfalls eine ovale Querschnittsform aufweist. Das jeweils andere Ende 122, welches zur Aufnahme der Gelenkkugel 30 dient,weist ebenfalls eine ovale Querschnittsform auf, jedoch auch andere Querschnittsformen sind möglich, wobei jeweils solche Querschnittsformen zu wählen sind, die der Querschnittsform der Konuseinstecköffnung in der Gelenkkugel 30 entsprechen.

Nach einer weiteren Ausführungsform der Erfindung ist der in den Hohlraum 11 des Stiels 10 eingezogene, rohrförmige Haltestutzen 15 unter Aufrechterhaltung einer sich über die gesamte Länge des Haltestutzens 15 erstreckenden gleichbleibenden Wandstärke zu seinem freien Ende 15a hin konisch auslaufend (Fig.2,3 und 4). Aufgrund dieser Ausgestaltung wird eine hohe Elastizität des Haltestutzens 15 zur Begrenzungswandung des Hohlraumes 11 erreicht.

## Patentansprüche

1. Femur-Hüftgelenkendoprothese mit einem zu seinem einen Ende hin konisch auslaufenden, anatomisch geformten Stiel (10) und mit einer an diesem gehaltenen Gelenkkugel (30) für eine Gelenkpfanne (40), dadurch gekennzeichnet, daß in dem der Gelenkkugel zugekehrten oberen Ende (10a) des Stiels (10) ein Hohlraum (11) ausgebildet ist,und daß der die obere Hohlraumöffnung (11a) begrenzende, umlaufende Rand (12) unter Ausbildung eines halsartigen und rohrförmigen Haltestutzens (15) in den Hohlraum (11) eingezogen ist, wobei der Haltestutzen (15) einen sich zu seinem freien Ende (15a) hin konisch auslaufenden Innenhohlraum (17) und einen ovalen Innenquerschnitt (16) für die Aufnahme einer Doppelkonus-Steckverbindung (20) aufweist, deren Doppelkonus (120) mindestens im Bereich des Haltestutzens (15) mit einer dessen Innenquerschnittsform entsprechenden Querschnittsform versehen ist.

2. Femur-Hüftgelenkendoprothese mit einem zu seinem einen Ende hin konisch auslaufenden, anatomisch geformten Stiel (10) und mit einer an diesem gehaltenen Gekenkkugel (30) für eine Gelenkpfanne (40), dadurch gekennzeichnet, daß in dem der Gelenkkugel zugekehrtes oberen Ende (10a) des Stiels (10) ein Hohlraum (11) ausgebildet ist, und daß der die obere Hohlraumöffnung (11a) begrenzende, umlaufende Rand (12) unter Ausbildung eines halsartigen und rohrförmigen Haltestutzens (15) in den Hohlraum (11) eingezogen ist, wobei der Haltestutzen (15) eine sich über die gesamte Länge des Haltestutzens (15) erstreckende gleichbleibende Wandstärke aufweist, zu seinem freien Ende hin (15a) konisch auslaufend ist und einen ovalen Querschnitt für die Aufnahme einer Doppelkonus-Steckverbindung (20) aufweist, deren Doppelkonus (120) mindestens im Bereich des Haltestutzens (15) mit einer dessen Innenquerschnittsform entsprechenden Querschnittsform versehen ist.

## Claims

1. A femur-hipjoint endoprosthesis having a stem (10) which is anatomically shaped and tapers conically towards its one end, and a joint bill (30) supported an the said stem for a joint socket (40), characterized in that a cavity (11) is formed in the top end (10a) of the stem (10) next the joint ball and that the circumferential edge (12) bounding the opening (11a) at the top of the cavity is drawn into the cavity (11) to form a tubular supporting connection (15) like a throat, the supporting connection (15) exhibiting an inner cavity (17) which tapers conically towards its free end (15a) and an oval inner cross-section (16) for receiving a double-conical plug connector (20) the double cone (120) of which is provided at least in the region of the supporting connection (15) with a cross-sectional shape which corresponds with the inner cross-sectional shape of the latter.

2. A femur-hipjoint endoprosthesis having a stem (10) which is anatomically shaped and tapers conically towards its one end, and a joint ball (30) supported on the said stem for a joint socket (40), characterized in that a cavity (11) is formed in the top end (10a) of the stem (10) next the joint ball and that the circumferential edge (12) bounding the opening (11a) at the top of the cavity is drawn into the cavity (11) to form a tubular supporting connection (15) like a throat, the wall of the supporting connection (15) exhibiting a constant thickness extending over the whole length of the supporting connection (15), whilst the latter tapers conically towards its free end (15a), exhibiting an oval cross-section for receiving a double-conical plug connector (20) the double cone (120) of which is provided at least in the region of the supporting connection (15) with a cross-sectional shape which corresponds with the inner cross-sectional shape of the latter.

## Revendications

1. Partie fémorale pour endoprothèse d'articulation de la hanche, comprenant une tige (10) de forme anatomique s'étendant sous forme conique en direction de l'une de ses extrémités et comprenant un condyle (30) qui est monté sur ladite tige (10) et qui correspond à une cavité glénoïde (40), caractérisée en ce qu'une cavité (11) est ménagée dans l'extrémité supérieure (10a) de la tige (10), ladite extrémité étant tournée vers le condyle, et en ce que le bord périphérique (12) qui délimite l'ouverture supérieure (11a) de la cavité est rentré dans la cavité (11) en formant un manchon de maintien (15) en forme de tube et semblable à un goulot, le manchon de maintien (15) présentant une cavité intérieure (17), qui s'étend sous forme conique en direction de l'extrémité libre (15a) dudit manchon de maintien (15), et une section intérieure ovale (16) pour recevoir un élément de liaison emboîtable biconique (20) dont le bicône (120) possède, au moins dans la zone du manchon de maintien (15), une forme en coupe transversale correspondant à la forme intérieure en coupe transversale dudit manchon de maintien.

2. Partie fémorale pour endoprothèse d'articulation de la hanche, comprenant une tige (10) de forme anatomique s'étendant sous forme conique en direction de l'une de ses extrémités et comprenant un condyle (30) qui est monté sur ladite tige (10) et qui correspond à une cavité glénoïde (40), caractérisée en ce qu'une cavité (11) est ménagée dans l'extrémité supérieure (10a) de la tige (10), ladite extrémité étant tournée vers le condyle, et en ce que le bord périphérique (12) qui délimite l'ouverture supérieure (11a) de la cavité est rentré dans la cavité (11) en formant un manchon de maintien (15) en forme de tube et semblable à un goulot, le manchon de maintien (15) présentant une épaisseur de paroi constante sur toute sa longueur (15), s'étendant sous forme conique en direction de son extrémité libre (15a) et présentant une section transversale ovale pour recevoir un élément de liaison emboîtable biconique (20) dont le bicône (120) possède, au moins dans la zone du manchon de maintien (15), une forme en coupe transversale correspondant à la forme intérieure en coupe transversale dudit manchon de maintien.
